Europäisches Patentamt

European Patent Office

Office européen des brevets

(11) Publication number: **0 398 362**
**A1**

(12) ## EUROPEAN PATENT APPLICATION

(21) Application number: 90109473.0

(22) Date of filing: 18.05.90

(51) Int. Cl.⁵: **C12N 9/72, C12N 15/58,**
**C12N 5/10**

A request for correction of page 5 of the description has been filed pursuant to Rule 88 EPC. A decision on the request will be taken during the proceedings before the Examining Division (Guidelines for Examination in the EPO, A-V, 2.2).

(30) Priority: **18.05.89 JP 126434/89**

(43) Date of publication of application:
**22.11.90 Bulletin 90/47**

(84) Designated Contracting States:
**BE CH DE ES FR GB IT LI NL SE**

(71) Applicant: **THE GREEN CROSS CORPORATION**
**3-3, Imabashi 1-chome Chuo-ku**
**Osaka-shi Osaka(JP)**

(72) Inventor: **Tanabe, Toshizumi, c/o The Green**
**Cross Corporation**
**Central Research Lab., 2-1180-1**
**Shodai-ohtani**
**Hirakata-shi, Osaka(JP)**
Inventor: **Amatsuji, Yasuo, c/o The Green**
**Cross Corporation**
**Central Research Lab., 2-1180-1**
**Shodai-ohtani**
**Hirakata-shi, Osaka(JP)**
Inventor: **Kasai, Shunji, c/o The Green Cross**
**Corporation**

Central Research Lab., 2-1180-1
Shodai-ohtani
Hirakata-shi, Osaka(JP)
Inventor: **Hirose, Masaaki, c/o The Green**
**Cross Corporation**
Central Research Lab., 2-1180-1
Shodai-ohtani
Hirakata-shi, Osaka(JP)
Inventor: **Morita, Masanori, c/o The Green**
**Cross Corporation**
Central Research Lab., 2-1180-1
Shodai-ohtani
Hirakata-shi, Osaka(JP)
Inventor: **Kawabe, Haruhide, c/o The Green**
**Cross Corporation**
Central Research Lab., 2-1180-1
Shodai-ohtani
Hirakata-shi, Osaka(JP)
Inventor: **Arimura, Hirofumi, c/o The Green**
**Cross Corporation**
Central Research Lab., 2-1180-1
Shodai-ohtani
Hirakata-shi, Osaka(JP)

(74) Representative: **Hansen, Bernd, Dr.**
**Dipl.-Chem. et al**
**Hoffmann, Eitle & Partner Patent- und**
**Rechtsanwälte Arabellastrasse 4**
**D-8000 München 81(DE)**

(54) **Human prourokinase variants, methods of producing same, DNA sequences, plasmids and hosts therefor.**

(57) A human prourokinase variant derived from human prourokinase by modifying the latter so that an amino acid sequence (I) of the formula

Asn-X-Y    (I)

wherein X is any amino acid residue and Y is Ser or Thr, occurs in the epidermal growth factor domain thereof which shows a half life in blood significantly longer as compared with known human PUK and urokinase and comparable to that of the human PUK variant deficient in the whole EGF domain.

# HUMAN PROUROKINASE VARIANTS, METHODS OF PRODUCING SAME, DNA SEQUENCES, PLASMIDS AND HOSTS THEREFOR

## FIELD OF THE INVENTION

This invention relates to human prourokinase (hereinafter referred to as "human PUK"), variants derived from human PUK by modifying its molecular structure, a method of producing the same, DNA saquences coding for said human PUK variants, plasmids with said DNA sequences inserted therein, and transformants harboring said plasmids. More specifically, the invention relates to a series of techniques for providing human PUK variants which comprise replacing, on the gene level, the amino acid sequence of a specific gene with another amino acid sequence for achieving modified carbohydrate chain binding on the protein level and then causing the so-modified gene to be expressed through application of the recombinant DNA technology.

## BACKGROUND OF THE INVENTION

There are two known species of plasminogen activating factor involved in fibrinolysis, urokinase (UK) and tissue plasminogen activator (t-PA). UK is a fibrinolytic enzyme, which thus far, has been purified from human urine and from the culture of human kidney cells. Recently, it has also become possible to produce UK by utilizing recombinant DNA technology (EP-A-154272 or JP-A-60-180591; the term "JP-A" as used herein means "an unexamined published Japanese patent application").

A serious side effect of UK is that when used in large doses, it may induce degradation and activation of various factors involved in coagulation and fibrinolysis, thus creating a tendency toward spontaneous bleeding. The present inventors found that the inactive precursor to human UK, prourokinase (hereinafter PUK) produced in human kidney cells (EP-A-139447 or JP-A-60-62981; J. Biol. Chem., 260, 12377 (1985)), unlike UK, will dissolve thrombi without creating a tendency toward spontaneous bleeding (Cell Struc. Func., 10, 151 (1985)).

Human PUK is composed of three functional domains, namely the epidermal growth factor (hereinafter abbreviated as "EGF") domain, the kringle domain and the enzyme activity domain (Hoppe-Seyler's Z. Physiol. Chem., 363 1155 (1982)).

For the three reasons mentioned below, it is presumed that a PUK-specific receptor is present on the liver cell and has an influence on the half-life in blood. The three reasons are: firstly, PUK intravenously administered to rats is metabolized mainly in the liver (Suyama et al.: "Fibrinolysis: Current Prospects", John Libbey & Company Ltd., London, pp. 35-43 (1988)); secondly, elimination of the EGF domain from t-PA, which is similar in structure to PUK, results in an increase in half-life in blood (Bowne et al.: J. Biol. Chem., 263, 1599 (1988)); and, thirdly, a UK-specific receptor is present on the human monocytic cell line U937 and the EGF domain of UK is involved in UK-receptor binding (Appella et al.: J. Biol. Chem., 262, 4437 (1987)).

The present inventors previously found that, in a test of half-life in blood using rats, a modified human PUK that lacked the EGF domain shows a half-life about three times longer than PUK with the EGF domain (EP-A-253241 or JP-A-63-146789). Deletion of any fairly large segment from a naturally occurring protein, however, may cause a great change in the structure of the original protein.

## SUMMARY OF THE INVENTION

Accordingly, the following are objectives of the present invention: 1) A human PUK variant having good affinity for fibrin and showing an increased half-life in blood comparable to that of the EGF domain-deficient human PUK variant while retaining substantially the same properties as those of human PUK by the least modification possible without deletion of the EGF domain; 2) A method of producing such human PUK variant; 3) A DNA sequence coding for said variant; 4) A plasmid with said DNA sequence inserted therein; and 5) A trasformant harboring said plasmid.

These and others objectives have been obtained by a modified human PUK variant including epidermal growth factor wherein an amino acid sequence (I) of the formula

Asn-X-Y    (I)

wherein X is any amino acid residue and Y is Ser or Thr, occurs in the EGF domain thereof. Another objective has been attained by a modified human PUK variant having a half life in blood longer than that of human PUK and comparable to that of the above-mentioned EGF domain-deficient PUK variant, good affinity for fibrin and, like human PUK or the EGF domain-deficient PUK variant, which does not create a tendency toward spontaneous bleeding.

Thus the invention provides a human PUK variant derived from human PUK by modifying the latter so that it has an amino acid sequence (I) in the EGF domain thereof, a DNA sequence coding for said human PUK variant, a plasmid with said DNA sequence inserted therein, a host transformed with said plasmid and a method of producing said human PUK variant.


## BRIEF DESCRIPTION OF THE DRAWINGS


Fig. 1 shows the amino acid sequence and the DNA base sequence of PUK.

Fig. 2 shows the processes for the construction of the plasmids pUH1, pUH2, pUH3, pUH4 and pUH7. In Fig. 2, stands for SV40 enhancer/promoter (E/P), for PUK cDNA and for poly A signal.

Fig. 3 shows the processes for the construction of the plasmids pHR22, pUH24 and pUH27. In Fig. 3, stands for SV40 E/P, for PUK cDNA, for poly A signal and for a synthetic DNA.

Fig. 4 shows the restriction enzyme cleavage map of the plasmid pSV-G1-preUK.


## DETAILED DESCRIPTION OF THE INVENTION


Among the 411 amino acid residues in human PUK, 49 amino acid residues from the N-terminal serine to the 49th amino acid residue threonine of the mature protein constitute the EGF domain (Riccio, A. et al.: Nucleic Acids Res., 13, 2759-2771 (1985)). The present invention provides a series of techniques for producing a human PUK variant so modified that it has an amino acid sequence (I) in the EGF domain so that a modifier carbohydrate chain can be bound thereto in the EGF domain.

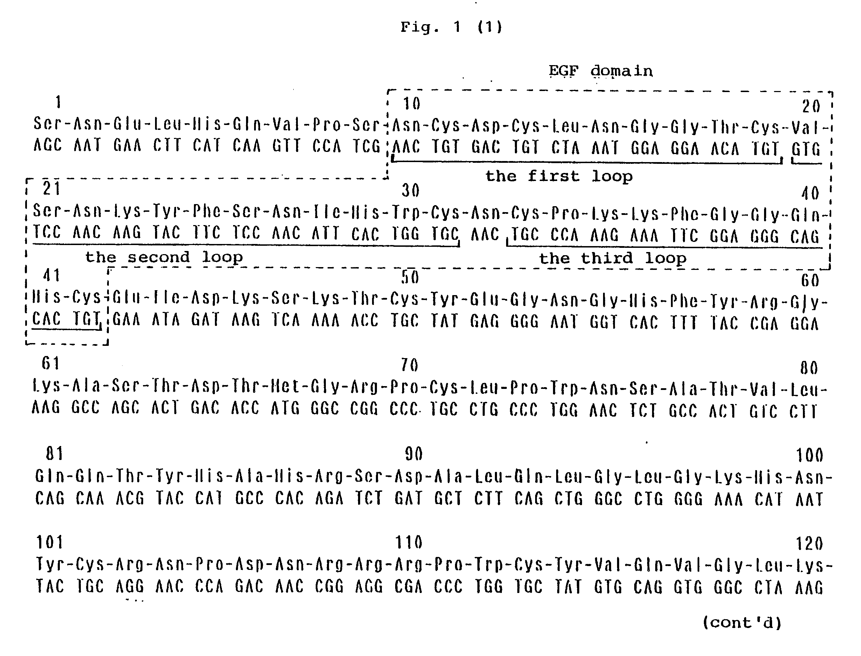

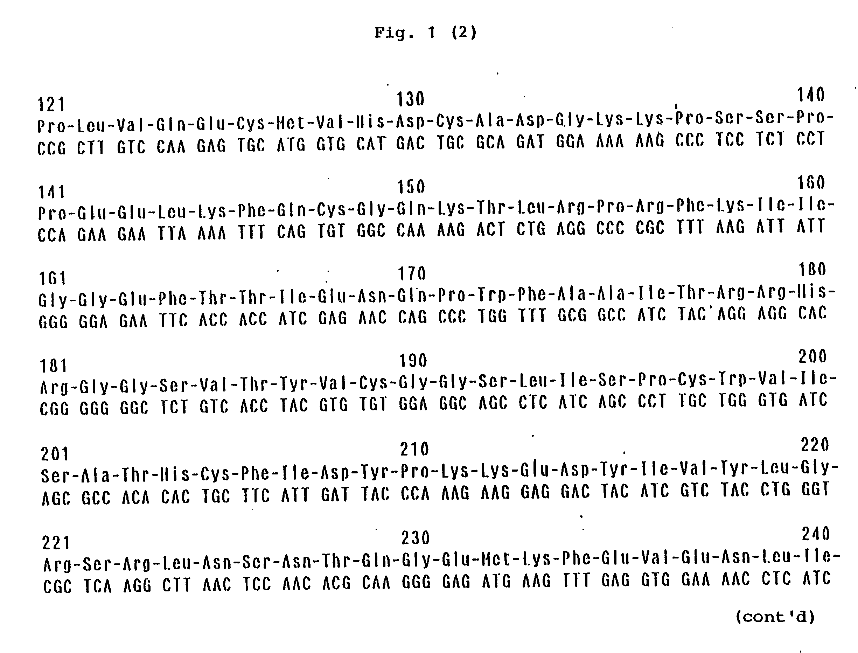

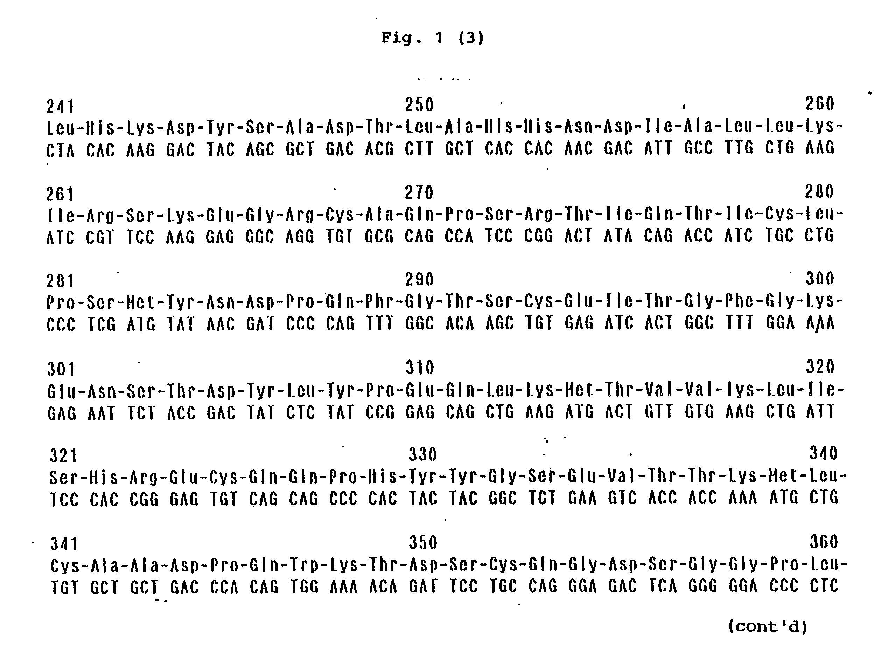

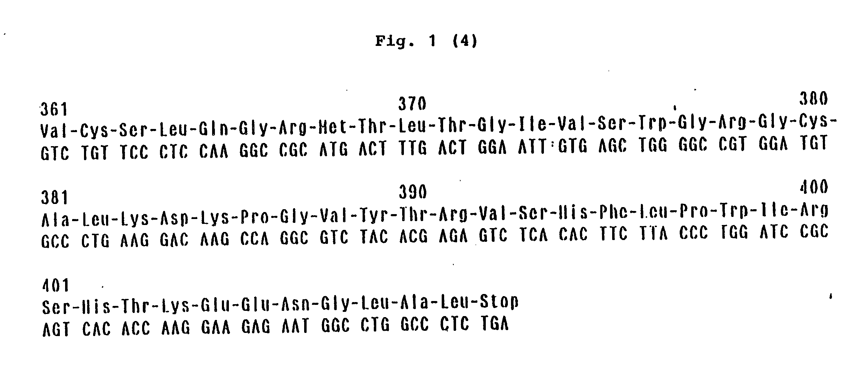

The EGF domain (Asn-10 to Cys-42) of PUK includes three loops; Asn-10 to Cys-19 form the first loop, Val-20 to Cys-31 form the second, and Cys-33 to Cys-42 form the third (Fig. 1). On the C-terminal side of the third loop, there is the kringle domain, which is said to contribute to PUK's affinity for fibrin. There are teachings suggesting that in an EGF-EGF receptor system and a UK-UK receptor system, the first and the second loop of the EGF domain function in the main in receptor binding of the EGF domain, the second loop contributes to the specificity of binding the receptor and the first loop stabilizes the bound receptor (J. Biol. Chem., 262, 4437-4440 (1987)). The third loop is adjacent to the kringle domain and its elimination may possibly result in great distortion of the kringle structure.

Investigations made by the present inventors have revealed that half life in blood of PUK varies depending on a carbohydrate chain type bound thereto. Namely, as compared with PUK having no carbohydrate chain, PUK having a complex type carbohydrate chain shows long half life in blood while PUK having a high mannose type carbohydrate chain shows short half life in blood.

The mode of protein-carbohydrate chain bonding includes N-glycosylation and O-glycosylation. Proteins having the amino acid sequence (I) are modified with a carbohydrate chain (N-glycosylation) in the process of their biosynthesis in mammalian cells. Thus, it was suggested that the complex type carbohydrate chain is effective in the sense that a carbohydrate chain of this type increases the stability of PUK in blood.

In the present invention, the amino acid residue represented by X in the amino acid sequence (I) is not limited to any particular species, selected from glycine, alanine, valine, leucine, isoleucine, serine, thereonine, cysteine, cystine, methionine, aspartic acid, aspargine, glutamic acid, glutamine, lysine, arginine, phenylalanine, tyrosine, hystidine, triptophane and proline. X may be any amino acid, either a naturally occurring one or a substituted amino acid.

In accordance with the invention, any site in the EGF domain may contain the amino acid sequence (I). Preferably, however, the second loop contains the sequence. Most preferably, Tyr-24 in the second loop is replaced with Ser-24 or Asn-24; or His-29 in the second loop is replaced with Ser-29, with a carbohydrate chain will be bound to Asn-22, Asn-24 or Asn-27.

Preferable examples of the PUK variants according to the present invention is shown in Table 1.

Table 1

| | Substituent amino acids | |
|---|---|---|
| PUK Variant gene-containing plasmid | 24th | 29th |
| pHR 22 | Ser (TCC) | His (CAC) |
| pHR 24 | Asn (ACC) | His (CAC) |
| pHR 27 | Tyr (TAC) | Ser (TCC) |
| human PUK | Tyr (TAC) | His (CAC) |
| Note: The parentheses are the corresponding codon for the amino acid. | | |

The amino acid sequence (I) is introduced into the EGF domain by deleting an optionally selected amino acid sequence and substituting a desired amino acid or amino acid sequence therefor. For the amino acid deletion and substitution, relevant techniques belonging to the technology of protein engineering can be widely utilized, for example site-directed deletion (Nucl. Acids Res., 11, 1645 (1983)), site-specific mutagenesis, and use of restriction enzymes and synthetic genes.

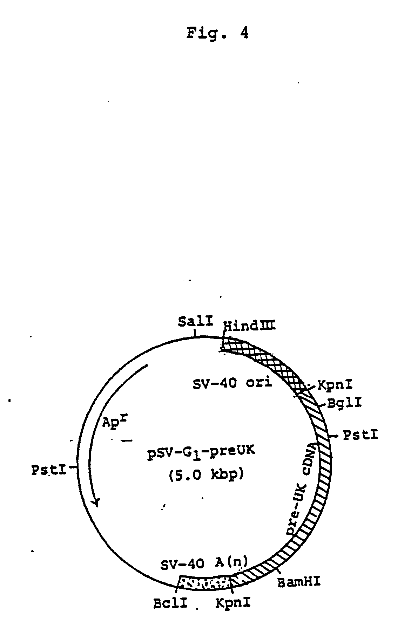

PUK cDNA can be obtained by the method described in EP-A-154272 or EP-A-253241. An example of the PUK cDNA-containing plasmid is pSV-G1-preUK (Fig. 4; EP-A-154272).

The PUK variant gene obtained from PUK that has undergone deletion/substituion treatment is inserted into an expression vector system to construct a host-vector system for expression. The host-vector system comprises a host and, in combination therewith, a plasmid vector containing a replicon and a regulatory sequence each derived from a species compatible with the host cells.

Vectors usable in the present invention contain a site for initiation of replication, a marker sequence enabling phenotypic selection of desired transformant cells and a promoter utilizable by a host and capable of causing a protein gene under the control thereof to be expressed. For instance, examples of suitable promoter for use in yeast vectors are the 3-phosphoglycerate kinase (PGK) promoter (Hitzeman et al.: J. Biol. Chem., 255, 2073 (1968)); and the promoters for other glycolytic system enzymes (Hess et al.: J. Adv. Enzyme Reg.: 7, 149 (1968)), Holland et al.: Biochemistry, 17, 4900 (1978)) such as enolase, glyceraldehyde-3-phosphate dehydrogenase (GAP-DH), hexokinase, pyruvate decarboxylase, phospho-fructokinase, glucose-6-phosphate isomerase, 3-phospho-glycerol phosphate mutase, pyruvate kinase, triose phosphate isomerase, phosphoglucose isomerase and glucokinase. Among them, a miniaturized GAP-DH promoter (JP-A-62-175180 or EP-A-218209) and the PGK promoter (Nucleic Acid Res., 10(8), 2625 (1982)) are preferred.

In constructing adequate expression vectors for the described invention, the transcription termination sequence (terminator) occurring in these genes is inserted into the vectors on the 3' side of the gene to be expressed so that poly(A) can be added to the mRNA and the transcription terminated. Also useful are promoters that are under transcriptional control depending on the growth conditions. Examples of such are the promoters for alcohol dehydrogenase 2, isocytochrome c, acid phosphatase, the enzymes involved in nitrogen metabolism, the above-mentioned glyceraldehyde-3-phosphate dehydrogenase, and the enzymes involved in the utilization of maltose or galactose.

Any plasmid vector containing a compatible yeast promoter, a replication origin and a terminator sequence may be used in the invention. Use is made of the Sacharomyces cerevisiae strains GRF18 (JP-A-59-48082 or EP-A-100561) and AH22 (ATCC 38626), for instance.

Plasmid having the replication origin of the plasmid pUB110 and the promoter, signal peptide gene and terminator of the α-amylase gene are useful for secretory expression vectors in Bacillus subtilis or Bacillus subtilis natto.

Vertebrate cells tissue culture is also useful (Tissue Culture, Academic Press, Kruse and Patterson, Editors (1973)). Examples of cell lines useful as hosts are VERO, HeLa cells, Chinese hamster ovary (CHO) cell lines, W138, BHK, COS-7, MDCK cell lines, C127, HKG, human kidney cell lines, etc. Expression vectors for use in these cells contain a replication origin, a promoter located upstream from the gene to be expressed, a ribosome binding site, an RNA splicing site, a poly(A) addition site and a terminator sequence.

When mammalian cells are used, the regulatory function of the expression vector is of viral origin. For example, the polyoma virus or adenovirus 2 promoter is a promoter in general use and the simian virus 40 (SV40) promoters may be used. The SV40 virus-derived early and late promoters are of particular use since they can be readily obtained from the virus as a fragment containing the replication origin of SV40 [Fiers et al.: Nature, 273, 113 (1978)]. This fragment is about 250 bp in size and covers the segment from the HindIII site to the BglI site in the replication origin can be used as well. Any promoter or regulatory sequence (enhancer) associated with the gene to be expressed can be used provided that it is compatible with the host.

Promoter-enhancers useful in expression vectors for use in animal cells, are the SV40 early or late gene promoter-enhancer, adenovirus major late promoter region, globulin enhancer-promoter region, RNA virus LTR, metallothionein promoter region, $\beta$-actin promoter. Useful replication origins are derived from SV40 or some other virus (e.g. polyoma virus, adenovirus, VSV, BPV) as inserted into the vector, or the replication mechanism of a host chromosome as such. If the vector can be incorporated into a host cell chromosome, the latter suffices. Furthermore, it is possible to use gene amplification systems utilizing the DHFR gene and other high-production systems.

Usable signal peptide in the present invention is one derived from the pUK gene, for example, derived from pSV-G1-preUK (EP-A-154272).

It is noted that the scope of the invention is by no means limited to those host cells, vectors and expression systems mentioned above as typical examples.

In a preferred embodiment of the invention, expression vectors for use in animal cells were constructed by insertion of a gene coding for a human PUK variant at a site downstream from the SV40 early promoter region.

For example, the expression vectors according to the present invention can be constructed as follows.

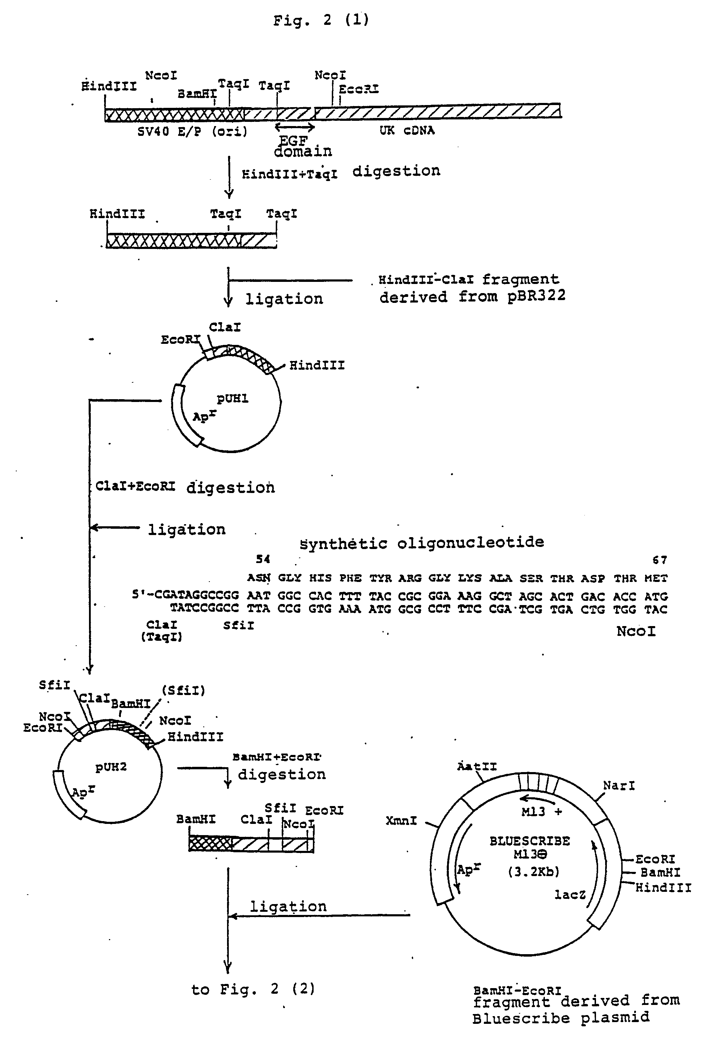

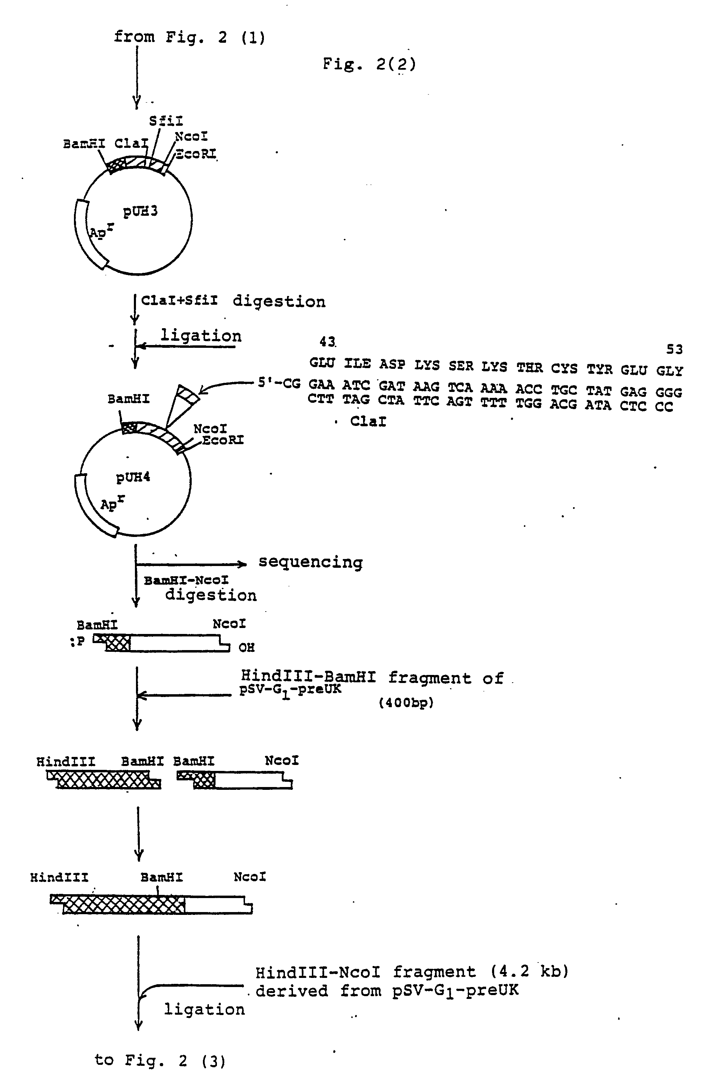

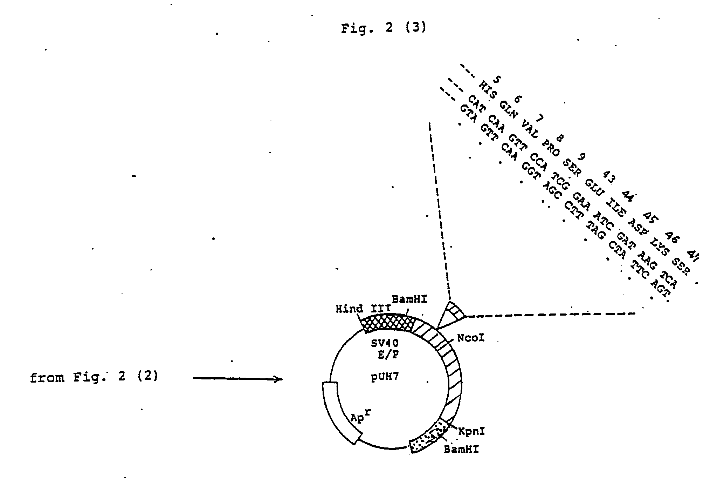

The human PUK expression vector pSV-G1-preUK (EP-A-154272) was digested with HindIII and TaqI and a DNA fragment containing a partial human PUK gene coding for the region from the first serine to 10th serine and SV40 enhancer-promoter was isolated and inserted into the plasmid pBR322 to give a plasmid, pUH1 (Fig. 2). Then, a synthetic gene coding for the region Asn-54 to Met-67 of human PUK and ending in a ClaI and an EcoRI site was prepared and inserted into the plasmid pUH1 to give a plasmid, pUH2. This synthetic gene contained a site recognizable by a peculiar restriction enzyme, SfiI.

The plasmid pUH2 was digested with BamI and EcoRI and the BamHI-EcoRI fragment containing only the SfiI site on the synthetic gene was inserted into the Bluescribe plasmid (Vector Cloning Systems) to give a plasmid, pUH3. A synthetic gene coding for Glu-43 to Gly-53 and ending in a ClaI and an SfiI site was inserted into said pUH3 at the SfiI-ClaI site thereof to give a plasmid, pUH4, containing a partial human PUK (Ser-1 to Ser-9 and Glu-43 to Met-67) gene, without the EGF domain region. Then, for incorporating such EGF domain region-free region into an expression vector, the BamHI-NcoI fragment of pUH4 was first ligated to the HindIII-BamHI fragment of pSV-G1-preUK. The resulting fragment was then inserted into pSV-G1-preUK at the HindIII-NcoI site thereof. Thus was constructed expression vector, pUH7 for EGF domain-deficient human PUK variant.

Then, 420 bp HindIII-BamHI DNA fragment of pUH7 and 3.5 kb HindIII-BamHI DNA fragment of pUH3 were ligated to each other to thereby obtain plasmid pTT03. This plasmid pTT03 contains SV40 enhancer-promoter region, the PUK signal sequence and a partial human PUK gene fragment (Ser-1 to Ser-9).

Separately, three synthetic DNAs coding for EGF domain (Asn-10 to Ile-44) with Ser 24 substituting for Tyr-24, Asn-24 substituting for Tyr-24, and Ser-29 substituting for His-29 were each prepared. These synthetic DNAs were each ligated to 3.9 kb DNA fragment of pTT03 previously digested with ClaI to obtain plasmids pHR21, pHR23 and pHR26. Then, 800 bp HindIII-ClaI DNA fragment of pHR21, pHR23 and pHR26 were each ligated to 4.2 kb DNA fragment of pUH7 previously digested with ClaI. Both ends of the resulting linear DNA fragments were rendered blunt and ligated to make circular DNA. Thus, PUK variant expression vectors, pHR22, pHR24 and pHR27 were obtaind.

They were introduced into CHO K1 cells for transformation. In this experimental system, clones (high production strains) producing PUK variants in amounts of 80, 340 and 37 IU/ml/3 days, respectively, were obtained.

The human PUk variants according to the invention can be purified by the known method of purifying human PUK (JP-A-60-62981 or EP-A-139447). In an embodiment of the invention, purification was effected by combined column chromatography using chelating Sepharose 6B, anti-UK IgG-Sepharose 4B and p-aminobenzamidine-Sepharose 6B. Chelating Sepharose 6B is particularly effective in crude purification, anti-UK IgG-Sepharose 4B in high-level purification, and p-aminobenzamidine-Sepharose 6B in removing contaminant active-form urokinase (UK).

Analysis of the products thus obtained revealed that the variants are comparable in PUK activity to the nonvariant form and that each of the PUK variants is a single-chain proenzyme having a molecular weight of 49,000 - 53,000 and entirely convertible to the active form upon plasmin treatment. Comparison of half-life in blood between the human PUK variants of the invention and human kidney cell-derived PUK (J. Biol. Chem., 260, 12377 (1985)) showed that the human prourokinase variants have a significantly prolonged half-life in blood.

The following Examples further illustrate the present invention in detail, but are not construed to limit the scope thereof.

In the Examples, synthetic DNAs were purified on modified polyacrylamide gels. The restriction enzymes, T4 DNA polymerase, enzyme kits (such as M13 sequencing kit and ligation kit), and Escherichia coli HB101 and JM109 competent cells used were obtained from Takara Shuzo. The calf intestine alkaline phosphatase used was a product of Boehringer Mannheim. Recombinant DNA techniques were applied as described in "Molecular Cloning", Cold Spring Harbor, New York, Cold spring Harbor Laboratory (1982).


## EXAMPLE 1


### (1) Construction of plasmid pUH1

The EGF domain-coding region (Asn-10-Cys-42) was eliminated from the human PUK gene contained in the human PUK expression vector plasmid pSV-G1-preUK (JP-A-60-180591 and EP-A-154272). pSV-G₁-preUK has a number of TagI sites and it is difficult to cleave the TagI site in the vicinity of the EGF domain region exclusively by one restriction enzyme treatment. Therefore, a DNA fragment containing the latter TagI site was excised. pSV-G1-preUK was digested with the restriction enzymes HindIII and BglII and the resulting DNA fragments were subjected to agarose gel electrophoresis. The gel portion containing an EGF domain-coding region-containing 1.1 kb DNA fragment was excised from the gel and the DNA was recovered. This fragment was then partially digested with TagI and the digest was subjected to polyacrylamide gel electrophoresis. The presence of the desired 760 bp band was confirmed and this DNA fragment was recovered.

Separately, the plasmid pBR322 was digested with HindIII and ClaI and a 4.3 kb DNA fragment was isolated. This DNA and the previously prepared 760bp HindIII-TagI DNA fragment were ligated together. This ligation resulted in conversion of the TagI site at the 3´ end of the 760 bp fragment to a ClaI site. The resulting ligation mixture was used to transform Escherichia coli HB101. The DNA prepared from each of 24 transformants thus obtained was digested with ClaI and NcoI. The desired plasmid pUH1 (Fig. 2 (1)) must be cleaved at one site only with each of ClaI and NcoI. Electrophoresis detected the desired plasmid in 6 clones.


### (2) Construction of plasmid pUH2

This plasmid pUH1 was digested with EcoRI and ClaI, the digest was subjected to agarose gel electrophoresis and a DNA fragment about 5.0 kb in size was recovered from the gel. Separately, the following two oligonucleotides were synthesized.

```
                            54
                Asn Gly His Phe Tyr Arg Gly Lys Ala Ser
     5'-CGATAGGCCGG  AAT GGC CAC TTT TAC CGC GGA AAG GCT AGC
          TATCCGGCC  TTA CCG GTG AAA ATG GCG CCT TTC CGA TGA
     ClaI (TaqI)  SfiI

                67
     Thr Asp Thr Met
     ACT GAC ACC ATG    56mer
     TGA CTG TGG TAC    54mer
              EcoRI
```

These synthetic oligonucleotides contained the region of Asn-54 to Met-67 of human PUK and ending in ClaI and EcoRI site. The annealing product of two synthetic oligonucleotides was ligated to the above 5.0 kb DNA fragment. The ligation mixture was used to transform Escherichia coli HB101. The DNA prepared from each of 20 transformant strains thus obtained was digested with NcoI, EcoRI and HindIII. The desired plasmid pUH2 (Fig. 2 (1)) must give a DNA fragment about 700 bp in size upon digestion with NcoI and a DNA fragment about 810 bp in size upon digestion with EcoRI and HindIII. As a result of electrophoresis, the desired plasmid was found in 4 clones.

(3) Construction of plasmid pUH3

Since, in pUH2, there are two SfiI sites in close proximity to each other, a BamHI-EcoRI fragment containing the synthetic gene-derived SfiI site was transferred to another vector. First, pUH2 was digested with BamHI and EcoRI and the digest was subjected to polyacrylamide gel electrophoresis. A 330 bp DNA fragment band was excised from the gel and the DNA was recovered by electroelution.

Separately, the plasmid Bluescribe (Vector Cloning Systems) was digested with BamHI and EcoRI and the thus-obtained DNA fragment about 3.1 kb in size was ligated to the previously prepared 330 bp BamHI-EcoRI fragment. The ligation mixture was used to transform Escherichia coli JM105. The plasmid DNA was prepared from each of 12 selected transformant strains was digested with SfiI and ClaI. The desired plasmid pUH3 (Fig. 2 (2)) must be cleaved only with one site with SfiI and with ClaI. As a result of electrophoresis, 8 clones were found to contain the desired plasmid.

(4) Construction of plasmid pUH4

pUH3 was digested with ClaI and SfiI, the digest was subjected to agarose gel electophoresis, and a DNA fragment about 3.1 kb in size was recovered by electroelution. Separately, the following two oligonucleotides were synthesized.

```
        43                                            53
        Glu Ile Asp Lys Ser Lys Thr Cys Tyr Glu Gly

    5'-CG GAA ATC GAT AAG TCA AAA ACC TGC TAT GAG GGG
       CTT TAG CTA TTC AGT TTT TGG ACG ATA CTC CC
```

These oligonucleotides contained the region of Glu-43 to Gly-53 of human PUK and ending in a ClaI and SfiI site. The annealing product of two synthetic oligonucleotides was ligated to the above 3.1 kb DNA fragment. The ligation mixture was used to transform Escherichia coli JM105. The plasmid DNA was prepared from each of 10 strains selected for the synthetic gene from among the transformants obtained and digested with BamHI and EcoRI. The desired plasmid pUH4 (Fig. 2 (2)) must give a 360 bp fragment upon the BamHI-EcoRI digestion. As a result of acrylamide gel electrophoresis, two clones harboring pUH4 were obtained.

The plasmid obtained in the above manner was used for the determination of the EGF domain region DNA base sequence. It was found that the respective contemplated deletion region (Asn-10 to Cys-42) had been deleted, without any change in the remaining amino acid sequence region.

(5) Construction of plasmid pUH7

Then, a partial gene covering the SV40 early gene promoter region to EGF domain-deficient human PUK variant gene was excised from the plasmid pUH4. pUH4 was cleaved with NcoI and then treated with calf intestine alkaline phosphatase for dephosphorylation at the 5' ends. The DNA was then digested with BamHI and the digest was subjected to polyacrylamide gel electrophoresis. A 360 bp DNA fragment-containing gel portion was excised and the DNA was recovered by electroelution. This DNA was further ligated to a separately prepared 400 bp BamHI-HindIII fragment of the plasmid pSV-G1-preUK. After ligation, the ligation product DNA was recovered by precipitation with ethanol.

Separately, pSV-G1-preUK was digested with HindIII and NcoI and the digest was subjected to electrophoresis. A portion containing a DNA fragment about 4.2 kb in size was excised from the gel and the DNA was recovered by electroelution. This 4.2 kb HindIII-NcoI DNA fragment was ligated to the above-mentioned ligation product. The resultant ligation mixture was used to transform Escherichia coli HB101. The plasmid DNA was extracted from each of 16 strains selected from among the transformant strains obtained and each DNA was digested with HindIII and with HindIII and BglII. The desired plasmid pUH7 (Fig. 2 (3)) must give a DNA fragment about 4.9 kb in size upon HindIII digestion and a fragment about 1.0 kb in size upon HindIII-BglII digestion. As a result of electrophoresis, 3 clones harboring the desired pUH7 were obtained. One of them was selected and this plasmid was prepared in large quantities.

(6) Construction of pTT03

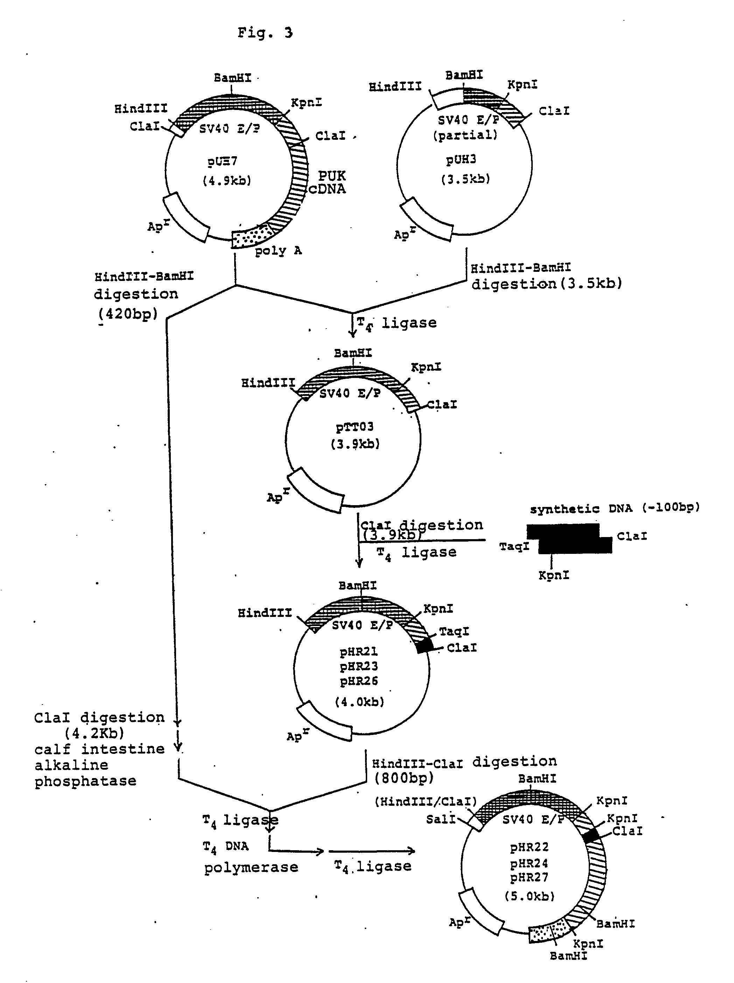

A plasmid, pTT03, containing the SV40 enhancer-promoter region, the PUK signal sequence and a gene coding for Ser-1 to Ser-9 of PUK was constructed. For obtaining 5 regions of the SV40 enhancer-promoter with a BamHI recognition site, pUH7 was digested with HindIII and BamHI and a 420 bp fragment was isolated and purified by agarose gel electophoresis. Separately, pUH3 was digested also with HindIII and BamHI and linearized 3.5 kb DNA fragment was isolated and purified by agarose gel electophoresis. This fragment was ligated to the above-mentioned 420 bp fragment with DNA ligase. A plasmid giving a 3.5 kb fragment and a 420 bp fragment upon digestion with HindIII and BamHI was named pTT03. Twelve transformants strains were examined and found all to be transformants harboring pTT03 (Fig. 3).

(7) Synthesis of oligonucleotides

Six single-stranded DNAs (DNA-1, DNA-2, DNA-3, DNA-4, DNA-5 and DNA-6) were prepared using an automatic DNA synthesizer (Applied Biosystems model 381A). Their base sequences are shown below.
For substituting Ser-24 for Tyr-24 (for carbohydrate addition on Asn-22):

```
DNA-1   5'-  C G  A A C   T G T   G A C   T G T   C T A   A A T
DNA-2   3'-       T T G   A C A   C T G   A C A   G A T   T T A

             A s n   C y s   A s p   C y s   L e u   A s n


                     KpnI
        G G A   G G T   A C C   T G T   G T G   T C C   A A C
        C C T   C C A   T G G   A C A   C A C   A G G   T T G

        G l y   G l y   T h r   C y s   V a l   S e r   A s n


                                         SspI
        A A G   T C C   T T C   T C C   A A T   A T T   C A C
        T T C   A G G   A A G   A G G   T T A   T A A   G T G

        L y s   S e r   P h e   S e r   A s n   I l e   H i s


        T G G   T G C   A A C   T G C   C C A   A A G   A A A
        A C C   A C G   T T G   A C G   G G T   T T C   T T T

        T r p   C y s   A s n   C y s   P r o   L y s   L y s
```

```
T T C   G G A   G G G   C A G   C A C   T G T   G A A
A A G   C C T   C C C   G T C   G T G   A C A   C T T
P h e   G l y   G l y   G l n   H i s   C y s   G l u


A T         -3'
T A G C.  -5'

I l e
```

For substituting Asn-24 for Tyr-24 (for carbohydrate addition on Asn-24):

```
DNA-3   5'-C G   A A C   T G T   G A C   T G T   C T A   A A T
DNA-4   3'-       T T G   A C A   C T G   A C A   G A T   T T A
                  A s n   C y s   A s p   C y s   L e u   A s n
```

```
                  KpnI
G G A   G G T   A C C   T G T   G T G   T C C   A A C
C C T   C C A   T G G   A C A   C A C   A G G   T T G
G l y   G l y   T h r   C y s   V a l   S e r   A s n
```

```
                                  SspI
A A G   A A C   T T C   T C C   A A T   A T T   C A C
T T C   T T G   A A G   A G G   T T A   T A A   G T G
L y s   A s n   P h e   S e r   A s n   I l e   H i s
```

```
T G G   T G C   A A C   T G C   C C A   A A G   A A A
A C C   A C G   T T G   A C G   G G T   T T C   T T T
T r p   C y s   A s n   C y s   P r o   L y s   L y s
```

```
T T C   G G A   G G G   C A G   C A C   T G T   G A A
A A G   C C T   C C C   G T C   G T G   A C A   C T T
P h e   G l y   G l y   G l n   H i s   C y s   G l u


A T-        3'
T A G C-   5'

I l e
```

For substituting Ser-29 for His-29 (for carbohydrate addtion on Asn-27):

```
DNA-5    5'-C G   A A C   T G T   G A C   T G T   C T A   A A T
DNA-6    3'-       T T G   A C A   C T G   A C A   G A T   T T A

                   A s n   C y s   A s p   C y s   L e u   A s n


                        KpnI
         G G A   G G T   A C C   T G T · G T G   T C C   A A C
         C C T   C C A   T G G   A C A   C A C   A G G   T T G

         G l y   G l y   T h r   C y s   V a l   S e r   A s n


                                         SspI
         A A G   T A C   T T C   T C C   A A T   A T T   T C C
         T T C   A T G   A A G   A G G   T T A   T A A   A G G

         L y s   T y r   P h e   S e r   A s n   I l e   S e r



         T G G   T G C   A A C   T G C   C C A   A A G   A A A
         A C C   A C G   T T G   A C G   G G T   T T C   T T T

         T r p   C y s   A s n   C y s   P r o   L y s   L y s


         T T C   G G A   G G G   C A G   C A C   T G T   G A A
         A A G   C C T   C C C   G T C   G T G   A C A   C T T

         P h e   G l y   G l y   G l n   H i s   C y s   G l u


         A T       -3'
         T A G C   -5'

         I l e
```

The first pair, DNA-1 and DNA-2, contains a DNA sequence coding for a PUK (Asn-10 to Ile-44) but differing from PUK in that the thyrosine residue in position 24 has been replaced with a serine residue; the second pair, DNA-3 and DNA-4, contains a DNA sequence coding for a PUK (Asn-10 to Ile-44) but differing from PUK in that the thyrosine residue in position 24 has been replaced with an asparagine residue; and the third pair, DNA-5 and DNA-6, contains a DNA sequence coding for a PUK (Asn-10 to Ile-44) but differing from PUK in that the histidine residue in position 29 has been replaced with a serine residue. In addition, in each pair, a KpnI recognition site has been introduced by converting GGAACA to GGTACC, and an SspI recognition site by converting AACATT to AATATT.

Following protective group elimination, a 64/100 portion of each synthesized oligonucleotide was purified by 12% acrylamide-urea gel electrophoresis. As a result, 15.9 µg of DNA-1, 22.0 µg of DNA-2, 19.0 µg of DNA-3, 21.1 µg of DNA-4, 8.8 µg of DNA-5 and 12.1 µg of DNA-6 were obtained each as a single-stranded DNA.


(8) Construction of pHR21, pHR23 and pHR26

pTT03 was digested with ClaI and a linearized 3.9 kb fragment was isolated and purified by agarose gel electrophoresis. This fragment was terminally dephosphorylated with calf intestine alkaline phosphatase. Separately, the oligonucleotides DNA-1 and DNA-2 synthesized as described above were subjected to kination and then to annealing, followed by ligation for joining to the dephosphorylated 3.9 kb fragment. A

10

plasmid, which gave a 800 bp DNA fragment upon digestion with HindIII and ClaI, was named pHR21. Screening of 12 transformants gave 6 transformants harboring pHR21. Similarly, a plasmid, pHR23, containing a synthetic DNA (DNA-3 and DNA-4) coding for a PUK variant with an asparagine residue substituting for the 24-position thyrosine residue of PUK and a plasmid, pHR26, containing a synthetic DNA (DNA-5 and DNA-6) coding for a PUK variant with a serine residue substituting for the 29-position histidine residue were constructed.

In the case of pHR23, 8 out of 12 transformants were transformants harboring the desired plasmid and, in the case of pHR26, 6 out of 12 transformants were the desired transformants. The synthetic DNA portion in each plasmid was confirmed also by base sequence determination.

## (9) Construction of pHR22, pHR24 and pHR27

Since pHR21, pHR23 and pHR26 have a DNA sequence coding for the first (serine) to 44th (isoleucine) residues of PUK, a DNA coding for the C-terminal side begining with the 45-position asparagine residue was jointed thereto for constructing pHR22, pHR24 and pHR27, respectively.

pUH7 was digested with ClaI and a 4.2 kb DNA fragment was isolated and purified by agarose gel electrophoresis. This fragment was dephosphorylated with calf intestine alkaline phosphatase and the digest was ligated to the 800 bp DNA fragment obtained by HindIII-ClaI digestion of pHR21 by utilizing the ClaI recognition site. Then, the HindIII recognition site and the ClaI recognition site (another end) were each rendered blunt with T4 DNA polymerase and ligation was again performed. Twenty four transformants were subjected to preliminary screening using the size of circular plasmid as index. Thus, comparison was made with a circular plasmid (pUH7) smaller by 100 bp than the desired plasmid. As a result, the plasmids in 5 out of the 24 transformants had the desired size. In this construction process, it was possible that the HindIII-ClaI fragment be inserted in the reverse direction. Therefore, plasmids giving a 900-bp DNA fragment upon KpnI digestion were then screened out. As a result, 4 out of the 5 plasmids were found to be the desired one. An Escherichia coli strain harboring this plasmid pHR22 has been deposited at the Institute for Fermentation, Osaka (17-85 2-chome, Juso-Honmachi, Yodogawa-ku, Osaka, Japan) since April 28, 1989 under the designation Escherichia coli HB101/pHR22 (deposit number IFO 14871).

pHR24 and pHR27 were constructed in the same manner. In both pHR24 and pHR27, only one of 24 plasmids screened was the desired plasmid.

## EXAMPLE 2

### Introduction into CHO cells of expression vector

The three human PUK variant expression vectors pHR22, pHR24, pHR27 were introduced into CHO K1 cells.

CHO K1 cells ($5 \times 10^6$ cells) in the logarithmic growth phase were cotransfected with the human PUK variant expression plasmid pHR22 (10 μg or 100 μg) and the plasmid pSV-G1-Neo (0.13 μg; EP-A-154272) to serve as a selective marker by the electroporation method. The cells were suspended in 50 ml of Ham's F12 medium (Nissui Pharmaceutical) supplemented with 10% fetal calf serum (MH01, Mitsubishi Kasei) and the suspension was distributed in 100-μl portion into wells of 96-well microplates. After incubation at 37 °C for 48 hours, G418 was added in a final concentration of 400 μg/ml. Thereafter, exchange of the selective medium was performed at 3- or 4-day intervals. When cells became almost confluent, the culture supernatant was assayed for PUK activity. Assuming that the human PUK activity of a variant in the culture supernatant was correlated with the activity of natural urokinase (UK), three days after medium exchange, the supernatant was collected and assayed for plasminogen activator (PA) activity by the fibrin agar plate method with a standard UK as a reference. Cell strains that had produced not less than 1 IU/ml of the human PUK variant were selected and cultured on a large scale, i.e. in 10 cm dishes, and activity assay was again carried out. pHR24 (10 μg or 100 μg) and pHR27 (10 μg or 100 μg) were also introduced into the same cells and the cell culture was carried out in the same manner. The results of the transfection are shown in Table 2.

Table 2:

| Transfection of CHO K1 cells with three human PUK variant expression plasmids;transduction efficiency and frequency of appearance of human PUK variant-producing strains | | |
|---|---|---|
| Expression plasmid | Transduction efficiency[a] | Number of human PUK variant-producing clones/number of G-418 resistant clones |
| pHR22/10 $\mu$g | $1.0 \times 10^{-4}$ | 9/49 (18%) |
| pHR24/10 $\mu$g | $1.3 \times 10^{-4}$ | 7/59 (12%) |
| pHR27/10 $\mu$g | $0.9 \times 10^{-4}$ | 2/43 ( 5%) |
| pHR22/100 $\mu$g | $0.34 \times 10^{-4}$ | 9/16 (56%) |
| pHR24/100 $\mu$g | $0.41 \times 10^{-4}$ | 13/19 (68%) |
| pHR27/100 $\mu$g | $0.22 \times 10^{-4}$ | 0/10 ( 0%) |

a) The value obtained by dividing the number of G-418 resistant clones by the number of CHO K1 cells was converted to a value per microgram of pSV-GI-neo. Since $5 \times 10^6$ cells were suspended in 50 ml and dis ributed in 0.1-ml portions into 360 wells, the number of CHO K1 cells used amounted to $3.6 \times 10^6$.

When 10 $\mu$g of each expression vector was used for the transduction, the transduction efficiency amounted to 0.9 to $1.3 \times 10^{-4}$ and there were no significant differences in the efficiency among the plasmids used. When the plasmids were used for transduction each in an amount of 100 $\mu$g, the efficiency values were 0.2 to $0.4 \times 10^{-4}$ and showed no significant differences. However, when the plasmids were used in an amount of 10 $\mu$g, the transduction efficiency was 3 to 4 times higher as compares with the cases where the plasmids were used in an amount of 100 $\mu$g.

When incorporated into cells, pSV-G1-neo used for the cotransfection gives G-418 resistance to the cells. When an equimolar amount of pSV-G1-neo is introduced into the same number of cells, the transduction efficiency must be constant. Since, however, as mentioned above, there was a great (10 times) difference in the quantity of the coexisting plasmid and the coexisting plasmid was present in a mole ratio of 80 or 800 to 1 relative to pSV-G1-neo when a pulsating 1,000-V electric field was applied, the expression plasmid might possibly have prevented pSV-G1-neo from being introduced into the cells. Thus, it is thought that the proportion of pSV-G1-neo relative to the coexisting plasmid might have caused the difference in transduction efficiency.

On the other hand, it is seen that the proportion of human PUK variant-producing clones among G-418 resistant clones was higher when 100 $\mu$g of the expression plasmid was used, that is when the molar proportion of the expression plasmid was higher, than when 10 $\mu$g of the expression plasmid was used. With the exception of pHR27, human PUK variant-producing strains can be obtained with a frequency of 10 to 20 % through the use of 10 $\mu$g of the expression plasmid or with a frequency as high as 50% or more through the use of 100 $\mu$g. In addition, it has become possible to isolate high production strains. The human PUK variant-producing clones obtained by transduction showed a distribution shown in Table 3 when grouped in terms of production capacity.

Table 3:

| Distribution of cells transfected with human PUK variant expression plasmids as grouped in terms of production on 96-well plates | | | | | |
|---|---|---|---|---|---|
| | Number of clones in a specific range of an PA activity (IU/ml) that the clones show | | | | |
| Plasmid introduced | 0 | 0-5 | 5-20 | 20-50 | 50- |
| pHR22/10 μg | 40 | 5 | 3 | 1 | 0 |
| pHR24/10 μg | 52 | 5 | 2 | 0 | 0 |
| pHR27/10 μg | 41 | 2 | 0 | 0 | 0 |
| pHR22/100 μg | 7 | 1 | 7 | 1 | 0 |
| pHR24/100 μg | 6 | 9 | 4 | 0 | 1 |
| pHR27/100 μg | 10 | 0 | 0 | 0 | 0 |

Since, however, medium exchange cannot be performed to a sufficient extent and the number of cells tends to vary widely under the growth conditions of 96-well plates, clones showing a production of 1 IU/ml or more were cultured on a larger scale, namely in 10-cm dishes, and activity comparison was made. The results are shown in Table 4.

Table 4:

| Distribution of cells transfected with human PUK variant expression plasmids as grouped in terms of production in 10 cm dishes | | | | |
|---|---|---|---|---|
| | Number of clones in an specific range of an PA activity (IU/ml) that the clones show | | | |
| Plasmid introduced | 0-5 | 5-20 | 20-50 | 50- |
| pHR22/10 μg | 2 | 1 | 2 | 1 |
| pHR24/10 μg | 1 | 4 | 0 | 0 |
| pHR27/10 μg | 0 | 0 | 1 | 0 |
| pHR22/100 μg | 0 | 5 | 3 | 0 |
| pHR24/100 μg | 3 | 7 | 3 | 1 |

Upon comparison between Table 2 and Table 3, it is seen that the PA activity of each clone is in general higher in Table 3. Based on this, it may be concluded that the growth conditions in 10-cm dishes are more favorable to human PUK variant production than those on 96-well plates.

Clones showing a human PUK variant production of not less than 20 IU/ml in 10-cm dishes are shown in Table 5 together with the activity data obtained when they were cultured on 96-well plates.

Table 5:

| Activities of human PUK variants in 10 cm dishes | | |
|---|---|---|
| | Activity (IU/ml) | |
| Clone | 10 cm dish | 96 well plate |
| pHR22(10)213 | 26 | 6 |
| pHR22(10)122 | 30 | 6 |
| pHR22(10)253 | 80 | 44 |
| pHR22(100)201 | 33 | 21 |
| pHR22(100)112 | 24 | 13 |
| pHR22(100)211 | 42 | 11 |
| pHR22(100)611 | 35 | 13 |
| pHR24(100)402 | 24 | 11 |
| pHR24(100)311 | 42 | 12 |
| pHR24(100)413 | 38 | 7 |
| pHR24(100)422 | 336 | 111 |
| pHR27(10)121 | 37 | 3 |

In this case, too, it can be seen that the growth conditions in 10-cm dishes are more preferable.

The introduction of the respective expression plasmids into CHO K1 cells gave the following high production clones: a 80 IU/ml clone upon introduction of 10 μg of pHR22; a 340 IU/ml clone upon introduction of 100 μg of pHR24; and a 37 IU/ml clone upon introduction of 10 μg of pHR27.

## EXAMPLE 3

### (1) Purification of human PUK variants

CHO K1 cells transfected with pHR22, CHO K1 cells transfected with pHR24 and CHO K1 cells transfected with pHR27 were used.

The culture supernatants of the above three transfectants were applied to a column of Chelating Sepharose 6B (Pharmacia) carrying zinc ions for adsorption of human PUK variant and the column was washed with 20 mM Tris-HCl buffer (pH 7.5) containing 10 KIU/ml aprotinin, and 1M sodium chloride, followed by elution with 20 mM Tris-HCl buffer (pH 7.5) containing 50 mM imidazole, 10 KIU/ml aprotinin and 1M sodium chloride.

The resulting eluate was applied to a column of benzamidine-Sepharose 6B (Pharmacia) equilibrated with 0.1 M phosphate buffer (pH 6.5) containing 0.5 M sodium chloride. Then, the column was washed with 0.1 phosphate buffer (pH 6.5) containing 10 KIU/ml aprotinin and 0.5 M sodium chloride and the fraction passed through the column was collected.

Formyl-Cellulofine (Pharmacia) to which anti-urine-derived urokinase antibody was bonded was equilibrated with 0.1 M phosphate buffer (pH 6.5) containing 0.5 M sodium chloride. The above-obtained fraction was applied thereto, the column was washed with the same buffer and then, elution was carried out with 0.2 M glycine-HCl buffer (pH 2.5) containing 0.5 M sodium chloride.

The eluate was further applied to a column of benzamidine-Sepharose 6B and the fraction passed through the column was collected.

This fraction was dialyzed against 0.1 M phosphate buffer (pH 6.2) to obtain purified human PUK variant.

The thus-obtained human PUK variants derived from pHR22, pHR24 and pHR27 are hereinafter referred to as Ser24-PUK, Asn24-PUK and Ser29-PUK, respectively. These three human PUK variants showed specific activity of 150,000 IU/mg protein when treated with plasmin.

### (2) Properties of human PUK variants

a) Molecular weight

A molecular weight of human PUK variants was determined by SDS-polyacrylamide gel electrophoresis (SDS-PAGE) in accordance with the method of Laemmli (Nature, 227, 680 (1970)).

189-350 IU of each human PUK variant was boiled at 100°C for 10 minutes in a reducing solution containing 2% 2-mercaptoethanol, 2% SDS, 10% glycerol and 50 mM Tris-HCl buffer (pH 6.8). Each reaction mixture was applied onto 10 to 20% gradient gel (Daiichi Kagaku Yakuhin) and electrophoresis was carried out at a constant current of 30 mA for 2 hours. The following molecular weight markers were used: phosphorylase b, 94,000; bovine serum albumin, 67,000; ovalbumin, 43,000; carbonic anhydrase, 30,000; trypsin inhibitor, 20,100; and $\alpha$-lactalbumin, 14,400 (Pharmacia).

After electrophoresis, the gel were stained by Coomassie Brilliant Blue R-250.

As a result, bands of Ser24-PUK and Asn24-PUK were found at 50KD and 52KD, respectively. With respect to Ser29-PUK, a smear-like band was observed at approximately 50 KD. Since the position of the bands of human PUK variants did not change under either reducing conditions or nonreducing conditions, it is suggested that human PUK variants according to the present invention have a single-stranded molecular structure.

b) Enzyme kinetics

Glt-Gly-Arg-MCA (hereinafter abbreviated as MCA), 7-amino-4-methyl Coumarin (hereinafter abbreviated as AMC) were commercially available from the Peptide Research Institute. The UK standard and plasmin used were products of The Green Cross Corporation.

The initial reaction rate was determined in the following manner.

A 100 $\mu$l portion of a 200 $\mu$l/ml solution of each human PUK variant (Ser24-PUK, Asn24-PUK and Ser29-PUK) was mixed with 100 $\mu$l of a 0.2 cu/ml solution of plasmin. As a buffer solution for human PUK variants and plasmin 0.1 M Tris-HCl buffer (pH 8.4) containing 0.1 M NaCl and 10% gelatin was used. The resulting mixture was incubated at 37°C for 10 minutes. Then, a 0.8 ml portion of 1.0, 0.5, 0.25, 0.125 or 0.625 mM solution of MCA previously maintained at 37°C was added to the mixture. After further incubation at 37°C for 3 minutes, 2 ml of a 15% acetic acid solution was added to the mixture to terminate the reaction. For comparison, naturally occurring human PUK (n-PUK; cf. EP-A-139447) was used. Fluorescence intensity of the resulting mixture was measured using a spectrophotofluorometer at an excitation wavelength of 370 nm and a fluorescence wavelength of 460 nm. The concentration of AMC produced by the enzyme reaction was calculated with taking the fluoresence intensity of 10 $\mu$M AMC as 100.

The kinetic constants, Km and Vmax, were calculated in accordance with the Lineweaver-Burk plot method (Segei, I.H. (1976) Biochemical Calculations, 2nd ed. John Wiley & Sons, Inc., New York). kcat was calculated by the following equation on the ground that 1 IU of UK corresponded to $1.33 \times 10^{-7}$ $\mu$mole.

$$\text{kcat} = \frac{\text{Vmax}}{1.33 \times 10^{-7}} \quad (\text{min.}^{-1})$$

The kinetic constants of each human PUK variant are shown in Table 6.

Table 6:

| Kinetic constant of human PUK variants | | | |
|---|---|---|---|
| Sample | KM ($\mu$M) | kcat $\times$ 10$^2$ (min.$^{-1}$) | kcat/Km |
| n-PUK | 355 | 84 | 2.4 |
| Ser24-PUK | 299 $\pm$ 30.1 | 81 $\pm$ 0 | 2.7 $\pm$ 0.3 |
| Asn24-PUK | 408 $\pm$ 73.9 | 104 $\pm$ 6 | 2.6 $\pm$ 0.3 |
| Ser29-PUK | 456 $\pm$ 115.1 | 100 $\pm$ 6 | 2.4 $\pm$ 0.7 |
| Note: The values shown in Table 6 are the mean $\pm$ standard deviation. The measurement was carried out once in the case of n-PUK and twice in the case of human PUK variants. | | | |

As shown in Table 6, there is no significant difference in kinetic constant between human PUK variants and natural human PUK.

c) Half-life in Blood

Half-lives in blood of n-PUK (natural: naturally occurring PUK derived from HGK cell; cf. EP-A-139447), n-PUK (recombinant: PUK produced by CHO K1 cells trasnformed with pSV-G1-preUK), $\Delta E_1 E_2 \Delta E_3$-PUK (human PUK variant deficient in the entire EGF domain), Ser24-PUK, Asn24-PUK and Ser29-PUK were examined.

The above six samples were labeled with $^{125}$I by the lactoperoxidase Enzymobeads (BIO-RAD) method. The radiochemical specific activity of $^{125}$I-PUK thus obtained was calculated from protein content and radioactivity determined by the absorbance at 280 nm. As a result, specific activity of each sample fell within the range from 11000 to 56000 cpm/IU.

The sample solution was adjusted with physiological saline so as to give a PUK concentration of 2 $\times$ 10$^4$ IU/ml (human albumin concentration: 5%) and was administered to Wister male rats (6-week-old) in a dose of 1 ml/kg through tail vein.

Rats were anesthetized with 35 mg/kg i.m. of ketamine hydrochloride (trade name: Ketalar® produced by Sankyo) and 1.5 g/kg i.m. of urethane (Nakarai Chemical) and fixed on the back. An atom venous catheter (3Fr) filled with a 3.8% solution of sodium citrate (The Green Cross Corporation) was inserted into left carotid artery. 1, 2, 3, 5, 7, 10, 15 and 20 minutes after the administration of samples, blood was collected from each rat using JMS 1 ml-volume of disposal syringe containing 30 $\mu$l of a 3.8% solution of sodium citrate so that blood was sucked up to the scale of 330 $\mu$l (300 $\mu$l in terms of blood). The sucked blood was shaken in the syringe and the whole sucked blood was measured for radioactivity using a $\gamma$-counter (COMPUGAMMA 1282 model, LKB WALLAC). After radioactivity of the blood was measured, the whole blood was centrifuged at 3000 rpm for 10 minutes to obtain 100 $\mu$l of plasma. The thus-obtained plasma was rapidly frozen on dry ice and stored at -20° C.

Time course of radioactivity of blood was measured and calculated in terms of % of dose. A half-life in blood was calculated by commercially available software.

A half-life in blood (T1/2$\alpha$) is shown in Table 7.

Table 7:

| T1/2α (min.) | |
|---|---|
| Sample | Radioactivity |
| n-PUK (Natural type) | 2.49 ± 0.17 |
| n-PUK (recombinant) | 2.25 ± 0.54 |
| $\Delta E_1 E_2 E_3$-PUK | 5.65 ± 0.66* |
| Ser24-PUK | 5.11 ± 0.10* |
| Asn24-PUK | 6.84 ± 1.14* |
| Ser29-PUK | 5.59 ± 0.99* |

Note: * Each value means the mean ± standard deviation (n = 8).

As shown in Table 7, there is a significant difference in half-life in blood such that human PUK variants according to the present invention show a half-life (in terms of radioactivity) about two to three times longer than n-PUKs ($p < 0.05$). Ser24-PUK, Asn24-PUK and Ser29-PUK showed T1/2α comparable to that of $\Delta E_1 E_2 E_3$-PUK.

As detailedly described hereinabove, the human PUK variants so modified according to the invention that they contain the amino acid sequence (I) in their EGF domain, in particular in the second loop thereof, undergo modificative carbohydrate chain addition (glycosylation) in the process of their metabolism in mammalian cells and the carbohydrate chains linked thereto inhibit the variants from interact with receptors on liver cells and exert an influence on the half life in blood (uptake by liver cells), increasing the half life. When the third loop is maintained, the kringle structure is not distorted; as a result, the affinity for fibrin is maintained.

The present invention makes it possible to provide human PUK variants which are of physiological significance as UK precursors and which show a half-life in blood significantly longer as compared with known human PUK and urokinase and comparable to that of the human PUK variant deficient in the whole EGF region.

Accordingly, the present invention provides more ideal fibrinolytic enzymes and is expected to be of great value in the field of therapeutics.

While the invention has been described in detail and with reference to specific embodiments thereof, it will be apparent to one skilled in the art that various changes and modifications can be made therein without departing from the spirit and scope thereof.

## Claims

1. A modified human prourokinase variant including an epidermal growth factor domain, wherein an amino acid sequence (I) of the formula
Asn-X-Y    (I)
wherein X is any amino acid residue and Y is Ser or Thr, occurs in the epidermal growth factor domain thereof.

2. A modified human prourokinase variant as claimed in Claim 1, wherein said amino acid sequence (I) occurs in the second loop in said epidermal growth factor domain.

3. A DNA sequence coding for the human prourokinase variant of Claim 1 or 2.

4. A plasmid containing the DNA sequence of Claim 3.

5. A host transformed with the plasmid of Claim 4.

6. A host as claimed in Claim 5, said host being an animal cell.

7. A method of producing a human prourokinase variant which comprises causing expression of a DNA sequence coding for a human prourokinase variant containing a modified epidermal growth factor domain in which an amino acid sequence (I) of the formula
Asn-X-Y    (I)
wherein X is any amino acid residue and Y is Ser or Thr, occurs in a host transformed with a plasmid containing said DNA sequence inserted therein.

8. A modified human prourokinase varient as claimed in claim 1, wherein Y is Ser.

9. A modified human prourokinase varient as claimed in claim 1, wherein Y is Thr.

10. A modified human prourokinase varient as claimed in claim 1, wherein within the epidermal growth factor demain, within the second loop thereof, Tyr-24 or His-29 is replaced.

11. A modified human prourokinase as varient as claimed in claim 10, wherein Tyr-24 is replaced by Ser-24 or Asn-24, or his-29 is replaced by Ser-29.

12. A DNA sequence coding for the human prourokinase varient of claim 8.

13. A DNA sequence coding for the human prourokinase varient of claim 9.

14. A DNA sequence coding for the human prourokinase varient of claim 10.

15. A DNA sequence coding for the human prourokinase varient of claim 11.

16. A plasmid containing the DNA sequence of claim 12.

17. A plasmid containing the DNA sequence of claim 13.

18. A plasmid containing the DNA sequence of claim 14.

19. A plasmid containing the DNA sequence of claim 15.

20. A host transformed with the plasmid of claim 16.

21. A host transformed with the plasmid of claim 17.

22. A host transformed with the plasmid of claim 18.

23. A host transformed with the plasmid of claim 19.

24. A plasmid pHR22.

Claims for the following Contracting State: ES

1. A method of producing a human prourokinase variant which comprises causing expression of a DNA sequence coding for a human prourokinase variant containing a modified epidermal growth factor domain in which an amino acid sequence (I) of the formula

Asn-X-Y    (I)

wherein X is any amino acid residue and Y is Ser or Thr, occurs in a host transformed with a plasmid containing said DNA sequence inserted therein.

2. A method of producing human prourokinase variant as claimed in Claim 1, wherein said amino acid sequence (I) occurs in the second loop in said epidermal growth factor domain.

3. A method of producing human prourokinase varient as claimed in claim 1, wherein Y is Ser.

4. A method of producing human prourokinase varient as claimed in claim 1, wherein Y is Thr.

5. A method of producing human prourokinase varient as claimed in claim 1, wherein within the epidermal growth factor demain, within the second loop thereof, Tyr-24 or His-29 is replaced.

6. A method of producing human prourokinase as varient as claimed in claim 5, wherein Tyr-24 is replaced by Ser-24 or Asn-24, or his-29 is replaced by Ser-29.

Fig. 1 (1)

EGF domain

```
 1                                           10                                                    20
Ser-Asn-Glu-Leu-His-Gln-Val-Pro-Ser Asn-Cys-Asp-Cys-Leu-Asn-Gly-Gly-Thr-Cys-Val-
AGC AAT GAA CTT CAT CAA GTT CCA TCG AAC TGT GAC TGT CTA AAT GGA GGA ACA TGT GTG
                                                   the first loop
 21                                          30                                                    40
Ser-Asn-Lys-Tyr-Phe-Ser-Asn-Ile-His-Trp-Cys-Asn-Cys-Pro-Lys-Lys-Phe-Gly-Gly-Gln-
TCC AAC AAG TAC TTC TCC AAC ATT CAC TGG TGC AAC TGC CCA AAG AAA TTC GGA GGG CAG
          the second loop                                    the third loop
 41                                          50                                                    60
His-Cys Glu-Ile-Asp-Lys-Ser-Lys-Thr-Cys-Tyr-Glu-Gly-Asn-Gly-His-Phe-Tyr-Arg-Gly-
CAC TGT GAA ATA GAT AAG TCA AAA ACC TGC TAT GAG GGG AAT GGT CAC TTT TAC CGA GGA

 61                                          70                                                    80
Lys-Ala-Ser-Thr-Asp-Thr-Met-Gly-Arg-Pro-Cys-Leu-Pro-Trp-Asn-Ser-Ala-Thr-Val-Leu-
AAG GCC AGC ACT GAC ACC ATG GGC CGG CCC TGC CTG CCC TGG AAC TCT GCC ACT GTC CTT

 81                                          90                                                   100
Gln-Gln-Thr-Tyr-His-Ala-His-Arg-Ser-Asp-Ala-Leu-Gln-Leu-Gly-Leu-Gly-Lys-His-Asn-
CAG CAA ACG TAC CAT GCC CAC AGA TCT GAT GCT CTT CAG CTG GGC CTG GGG AAA CAT AAT

101                                         110                                                  120
Tyr-Cys-Arg-Asn-Pro-Asp-Asn-Arg-Arg-Arg-Pro-Trp-Cys-Tyr-Val-Gln-Val-Gly-Leu-Lys-
TAC TGC AGG AAC CCA GAC AAC CGG AGG CGA CCC TGG TGC TAT GTG CAG GTG GGC CTA AAG
```

(cont'd)

EP 0 398 362 A1

Fig. 1 (2)

121                                     130                                     140
Pro-Leu-Val-Gln-Glu-Cys-Met-Val-His-Asp-Cys-Ala-Asp-Gly-Lys-Lys-Pro-Ser-Ser-Pro-
CCG CTT GTC CAA GAG TGC ATG GTG CAT GAC TGC GCA GAT GGA AAA AAG CCC TCC TCT CCT

141                                     150                                     160
Pro-Glu-Glu-Leu-Lys-Phe-Gln-Cys-Gly-Gln-Lys-Thr-Leu-Arg-Pro-Arg-Phe-Lys-Ile-Ile-
CCA GAA GAA TTA AAA TTT CAG TGT GGC CAA AAG ACT CTG AGG CCC CGC TTT AAG ATT ATT

161                                     170                                     180
Gly-Gly-Glu-Phe-Thr-Thr-Ile-Glu-Asn-Gln-Pro-Trp-Phe-Ala-Ala-Ile-Thr-Arg-Arg-His-
GGG GGA GAA TTC ACC ACC ATC GAG AAC CAG CCC TGG TTT GCG GCC ATC TAC AGG AGG CAC

181                                     190                                     200
Arg-Gly-Gly-Ser-Val-Thr-Tyr-Val-Cys-Gly-Gly-Ser-Leu-Ile-Ser-Pro-Cys-Trp-Val-Ile-
CGG GGG GGC TCT GTC ACC TAC GTG TGT GGA GGC AGC CTC ATC AGC CCT TGC TGG GTG ATC

201                                     210                                     220
Ser-Ala-Thr-His-Cys-Phe-Ile-Asp-Tyr-Pro-Lys-Lys-Glu-Asp-Tyr-Ile-Val-Tyr-Leu-Gly-
AGC GCC ACA CAC TGC TTC ATT GAT TAC CCA AAG AAG GAG GAC TAC ATC GTC TAC CTG GGT

221                                     230                                     240
Arg-Ser-Arg-Leu-Asn-Ser-Asn-Thr-Gln-Gly-Glu-Met-Lys-Phe-Glu-Val-Glu-Asn-Leu-Ile-
CGC TCA AGG CTT AAC TCC AAC ACG CAA GGG GAG ATG AAG TTT GAG GTG GAA AAC CTC ATC

(cont'd)

EP 0 398 362 A1

Fig. 1 (3)

241                             250                             260

Leu-His-Lys-Asp-Tyr-Ser-Ala-Asp-Thr-Leu-Ala-His-His-Asn-Asp-Ile-Ala-Leu-Leu-Lys-

CTA CAC AAG GAC TAC AGC GCT GAC ACG CTT GCT CAC CAC AAC GAC ATT GCC TTG CTG AAG

261                             270                             280

Ile-Arg-Ser-Lys-Glu-Gly-Arg-Cys-Ala-Gln-Pro-Ser-Arg-Thr-Ile-Gln-Thr-Ile-Cys-Leu-

ATC CGT TCC AAG GAG GGC AGG TGT GCG CAG CCA TCC CGG ACT ATA CAG ACC ATC TGC CTG

281                             290                             300

Pro-Ser-Met-Tyr-Asn-Asp-Pro-Gln-Phr-Gly-Thr-Ser-Cys-Glu-Ile-Thr-Gly-Phe-Gly-Lys-

CCC TCG ATG TAT AAC GAT CCC CAG TTT GGC ACA AGC TGT GAG ATC ACT GGC TTT GGA AAA

301                             310                             320

Glu-Asn-Ser-Thr-Asp-Tyr-Leu-Tyr-Pro-Glu-Gln-Leu-Lys-Met-Thr-Val-Val-lys-Leu-Ile-

GAG AAT TCT ACC GAC TAT CTC TAT CCG GAG CAG CTG AAG ATG ACT GTT GTG AAG CTG ATT

321                             330                             340

Ser-His-Arg-Glu-Cys-Gln-Gln-Pro-His-Tyr-Tyr-Gly-Ser-Glu-Val-Thr-Thr-Lys-Met-Leu-

TCC CAC CGG GAG TGT CAG CAG CCC CAC TAC TAC GGC TCT GAA GTC ACC ACC AAA ATG CTG

341                             350                           360

Cys-Ala-Ala-Asp-Pro-Gln-Trp-Lys-Thr-Asp-Ser-Cys-Gln-Gly-Asp-Ser-Gly-Gly-Pro-Leu-

TGT GCT GCT GAC CCA CAG TGG AAA ACA GAT TCC TGC CAG GGA GAC TCA GGG GGA CCC CTC

(cont'd)

EP 0 398 362 A1

## Fig. 1 (4)

```
361                                   370                              380
Val-Cys-Ser-Leu-Gln-Gly-Arg-Met-Thr-Leu-Thr-Gly-Ile-Val-Ser-Trp-Gly-Arg-Gly-Cys-
GTC TGT TCC CTC CAA GGC CGC ATG ACT TTG ACT GGA ATT GTG AGC TGG GGC CGT GGA TGT

381                                   390                              400
Ala-Leu-Lys-Asp-Lys-Pro-Gly-Val-Tyr-Thr-Arg-Val-Ser-His-Phe-Leu-Pro-Trp-Ile-Arg
GCC CTG AAG GAC AAG CCA GGC GTC TAC ACG AGA GTC TCA CAC TTC TTA CCC TGG ATC CGC

401
Ser-His-Thr-Lys-Glu-Glu-Asn-Gly-Leu-Ala-Leu-Stop
AGT CAC ACC AAG GAA GAG AAT GGC CTG GCC CTC TGA
```

EP 0 398 362 A1

## Fig. 2 (1)

HindIII + TaqI digestion

ligation

HindIII-ClaI fragment derived from pBR322

ClaI + EcoRI digestion

ligation

synthetic oligonucleotide

```
            54                                                      67
            ASN GLY HIS PHE TYR ARG GLY LYS ALA SER THR ASP THR MET
5'-CGATAGGCCGG AAT GGC CAC TTT TAC CGC GGA AAG GCT AGC ACT GAC ACC ATG
   TATCCGGCC TTA CCG GTG AAA ATG GCG CCT TTC CGA TCG TGA CTG TGG TAC
   ClaI        SfiI                                              NcoI
   (TaqI)
```

BamHI + EcoRI digestion

ligation

BamHI-EcoRI fragment derived from Bluescribe plasmid

to Fig. 2 (2)

from Fig. 2 (1)

Fig. 2(2)

ClaI+SfiI digestion

ligation

```
        43                                          53
        GLU ILE ASP LYS SER LYS THR CYS TYR GLU GLY
5'-CG GAA ATC GAT AAG TCA AAA ACC TGC TAT GAG GGG
   CTT TAG CTA TTC AGT TTT TGG ACG ATA CTC CC
 · ClaI
```

sequencing

BamHI-NcoI digestion

HindIII-BamHI fragment of pSV-G₁-preUK (400bp)

HindIII-NcoI fragment (4.2 kb) derived from pSV-G₁-preUK

ligation

to Fig. 2 (3)

Fig. 2 (3)

from Fig. 2 (2) ⟶

Labels on plasmid map: Hind III, BamHI, SV40 E/P, pUH7, NcoI, Ap^r, KpnI, BamHI

Sequence (along dashed lines):
```
        5   6   7   8   9   43  44  45  46  47
    HIS GLN VAL PRO SER GLU ILE ASP LYS SER
    CAT CAA GTT CCA TCG GAA ATC GAT AAG TCA
    GTA GTT CAA GGT AGC CTT TAG CTA TTC AGT
```

Fig. 3

Fig. 4

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| P,X | WO-A-8 911 531 (GENENTECH INC.)<br>* Claims; page 14, line 34 – page 15, line 13 * | 1-23 | C 12 N 9/72<br>C 12 N 15/58<br>C 12 N 5/10 |
| | | | **TECHNICAL FIELDS SEARCHED (Int. Cl.5)**<br><br>C 12 N |

The present search report has been drawn up for all claims

| Place of search | Date of completion of the search | Examiner |
|---|---|---|
| THE HAGUE | 03-08-1990 | HUBER A. |